(19) Europäisches Patentamt
European Patent Office
Office européen des brevets

(11) **EP 2 826 421 B1**

(12) **EUROPEAN PATENT SPECIFICATION**

(45) Date of publication and mention
of the grant of the patent:
**14.09.2016 Bulletin 2016/37**

(51) Int Cl.:
*A61B 5/11* (2006.01)   *A61B 7/00* (2006.01)
*A61B 5/00* (2006.01)

(21) Application number: **12871029.0**

(22) Date of filing: **16.03.2012**

(86) International application number:
**PCT/JP2012/056944**

(87) International publication number:
**WO 2013/136530 (19.09.2013 Gazette 2013/38)**

(54) **TREMOR-DETECTING DEVICE AND TREMOR-DETECTING METHOD**

TREMORERKENNUNGSVORRICHTUNG UND TREMORERKENNUNGSVERFAHREN

DISPOSITIF DE DÉTECTION DE TREMBLEMENTS ET PROCÉDÉ DE DÉTECTION DE TREMBLEMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**21.01.2015 Bulletin 2015/04**

(73) Proprietor: **FUJITSU LIMITED
Kawasaki-shi
Kanagawa 211-8588 (JP)**

(72) Inventor: **KASAMA, Kouichirou
Kawasaki-shi
Kanagawa 211-8588 (JP)**

(74) Representative: **Schultes, Stephan
Haseltine Lake LLP
300 High Holborn
London WC1V 7JH (GB)**

(56) References cited:
**GB-A- 2 438 167      JP-A- S63 212 330
JP-A- 2000 217 802    JP-A- 2005 270 570
US-A1- 2006 251 334**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD

The embodiments discussed herein are related to a fluctuation detection device and a fluctuation detection method that detect fluctuation of a body or the like.

BACKGROUND

Conventionally, there has been a technique that detects movement of the mobile terminal by using an acceleration sensor, a gyro sensor, an infrared sensor, etc. and thereby determines whether the body of a user carrying a mobile terminal is fluctuating.

As a technique related to the above technique, a method is known, among other methods, in which marking points are attached to joints such as a shoulder, the waste, etc. of a user and these points are made to reflect light beams such as infrared rays so that the movement of the user is detected.

Also, an evaluation method is known in which horizontal plane acceleration accumulated value $\sum\Delta i$ of horizontal plane acceleration $\Delta i(=\sqrt{(xi\times xi+yi\times yi)})$ that is calculated from measurement value xi of the forward-backward acceleration and measurement value yi of the right-left acceleration of a subject obtained by using an acceleration meter is used as an index of the fluctuation evaluation of the subject.

Patent Document 1: Japanese Laid-open Patent Publication No. 2004-344468
Patent Document 2: Japanese Laid-open Patent Publication No. 2008-073267

GB2438167 discloses a system and method for monitoring a person's balance, for example when performing a posturography assessment following a stroke, in which a unit bearing two or more light emitting devices in a predetermined spatial arrangement is attached to a person. A simple video capture device such as a webcam may then be used to image the light emitting devices while the person performs balance assessing exercises. By measuring movement of the light emitting devices, for example relative to each other or to some fixed datum, it is possible to obtain an objective measure of balance. The unit maybe attached to the person by a belt, strap or clip. The light emitting devices each emit light having a different characteristic from the other light emitting devices, this maybe the colour or wavelength of the emitted light.

US2006/0251334 discloses a balance diagnostic apparatus and system which is given a portable size and weight and also expands the places of use and methods of use to a large range, thus achieving an environment where anyone can undergo diagnosis of balance disorders at any time or any place. At least motion sensor means and motion storage means that temporarily stores signals that represent motion from the motion sensor means are worn on the body of the user. Moreover, once the motion situation is stored in the motion storage means, the input of the motion diagnosis means is connected to the output of the motion storage means to obtain the output of diagnostic results with this balance function diagnostic system.

SUMMARY

Problem to be Solved by the Invention

However, it has been difficult for an acceleration sensor or a gyro sensor to detect minute body fluctuation that falls below the resolution of the sensor. Also, it has been difficult to measure minute fluctuation because of body activities such as breathing. Hereinafter, minute body fluctuation that falls below the resolution of a sensor such as an acceleration sensor or a gyro sensor is simply referred to as "fluctuation".

According to one aspect, it is an object to provide a fluctuation detection device that detects body fluctuation more accurately.

Means to Solve the Problem

According to one aspect of the present invention, there is provided a fluctuation detection device configured to detect fluctuation of the body of a user, the fluctuation detection device comprising: a measurement unit configured to measure a first distance that is a distance to a still object existing in a first direction and a second distance that is a distance to a still object existing in a second direction different from the first direction; a calculation unit configured to calculate a first fluctuation amount that is a changing amount of the first distance and a second fluctuation amount that is a changing amount of the second distance; and a removal unit configured to remove a changing amount of the first distance caused by breathing of the user from the first fluctuation amount and removes a changing amount of the second distance caused by the breathing from the second fluctuation amount, when an amplitude value of a particular frequency band from among frequency bands obtained by performing Fourier transform on sound data of the breathing of the user is equal to or greater than a fixed value.

According to another aspect of the present invention, there is provided a fluctuation detection method that detects fluctuation of a body of a user, the fluctuation detection method comprising: measuring a first distance that is a distance to a still object existing in a first direction and a second distance that is a distance to a still object existing in a second direction different from the first direction; calculating a first fluctuation amount that is a changing amount of the first distance and a second fluctuation amount that is a changing amount of the second distance; detecting breathing of the user; and removing a changing amount of the first distance caused by the breathing from the first fluctuation amount and removing a changing amount of the second distance caused by the breathing from the second fluctuation amount when breathing of the user is detected during the measurement, when an amplitude value of a particular frequency band from among frequency bands obtained by performing Fourier transform on sound data of the breathing of the user is equal to or greater than a fixed value.

Effect of the Invention

According to an aspect, it is possible to provide a fluctuation detection device that detects body fluctuation more accurately.

BRIEF DESCRIPTION OF DRAWINGS

FIG. 1 illustrates an outline of a fluctuation detection device 100;
FIG. 2 illustrates an outline of a sensor included in a fluctuation detection device 200;
FIG. 3 illustrates an example of mounting the fluctuation detection device 200;
FIG. 4 illustrates an outline of fluctuation detection performed by the fluctuation detection device 200;
FIG. 5 illustrates a configuration example of the fluctuation detection device 200;
FIG. 6 illustrates an example of a shared information table 600;
FIG. 7 illustrates an example of an information management table 700;
FIG. 8 illustrates an example of a first determination table 800;
FIG. 9 illustrates an example of a second determination table 900;
FIG. 10 illustrates an example of a total determination table 1000;
FIG. 11 is a flowchart illustrating processes performed by the fluctuation detection device 200;
FIG. 12 is a flowchart illustrating a process of thread 1 executed by the fluctuation detection device 200;
FIG. 13 is a flowchart illustrating processes of thread 2 executed by the fluctuation detection device 200;
FIG. 14 is a flowchart illustrating processes of thread 3 executed by the fluctuation detection device 200;
FIG. 15 illustrates an example of detecting fluctuation of the body of a user 300 by using the fluctuation detection device 200; and
FIG. 16 illustrates an example of detecting fluctuation of the body of the user 300 by using the fluctuation detection device 200.

DESCRIPTION OF EMBODIMENTS

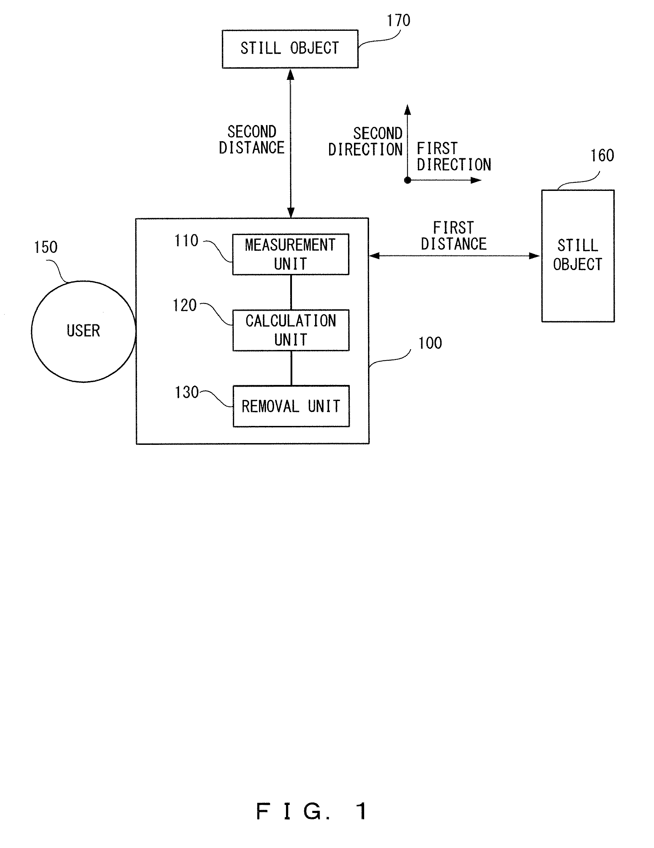

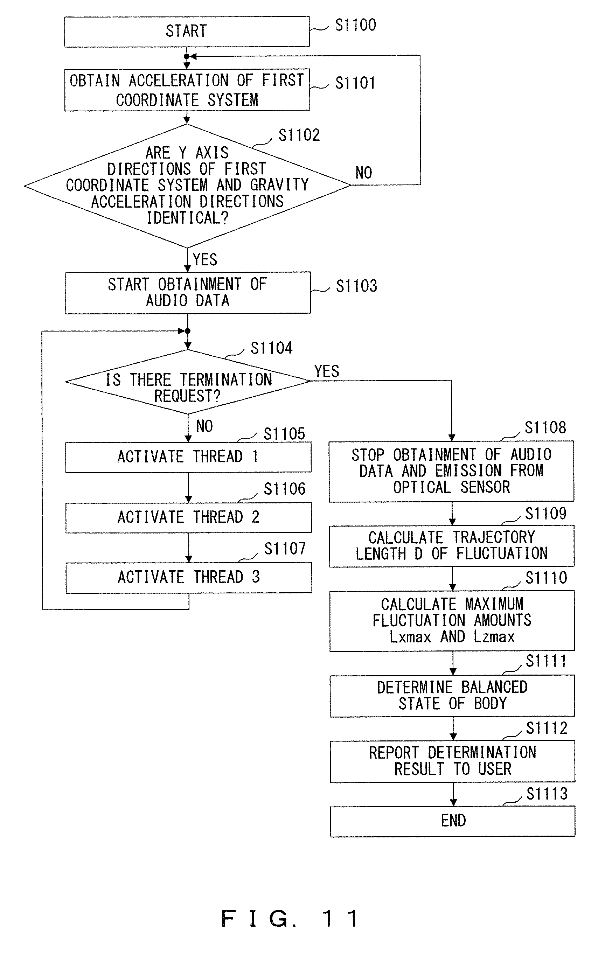

Hereinafter, an example of an embodiment of the present invention will be explained by referring to FIG. 1 through FIG. 16. Note that the embodiments below are just exemplary and are not intended to exclude various alterations or applications of techniques that are not described hereinbelow. In other words, the present embodiments can be implemented in various alterations such as combining the respective examples. Also, the process orders illustrated in the form of the flowcharts in FIG. 11, FIG. 12, FIG. 13 and FIG. 14 are not intended to limit the orders of the processes. Accordingly, it is natural that the orders of the processes may be changed when it is possible.

<Example>

FIG. 1 illustrates an outline of a fluctuation detection device 100 according to an example. The fluctuation detection device 100 includes a distance measurement unit 110, a calculation unit 120 and a removal unit 130. The fluctuation detection device 100 can be used by being attached to a user.

The distance measurement unit 110 measures a first distance, which is a distance from a still object 160 existing in the first direction, and a second distance, which is a distance from a still object 170 existing in the second direction that is different from the first direction. The distance measurement unit 110 measures the first and second distances at consistent intervals e.g., at the intervals of 0.2ms.

The still objects 160 and 170 are for example walls or the like. Also, it is desirable that the first and second directions be orthogonal to each other as illustrated in FIG. 1, however, the scope of the present invention is not limited to this.

The calculation unit 120 calculates the first fluctuation amount from variation amounts of the first distances measured by the distance measurement unit 110. Similarly, the calculation unit 120 calculates the second fluctuation amount from variation amounts of the second distances measured by the distance measurement unit 110.

A removal unit 140 removes, from the first fluctuation amount, the variation amounts of the first distances that are caused by the breathing of a user 150 and removes, from the second fluctuation amount, the variation amounts of the second distances that are caused by the breathing.

As described above, the fluctuation detection device 100 removes, from the first fluctuation amount, the variation amounts of the first distances that are caused by the breathing of the user 150. Similarly, the fluctuation detection device 100 removes, from the second fluctuation amount, the variation amounts of the second distances that are caused by the breathing of the user 150. Thereby, the fluctuation detection device 100 can remove measurement errors in the first and second distances that are caused by the widening of the body, occurring due to the breathing, of the user 150 wearing the fluctuation detection device 100. As a result of this, the fluctuation detection device 100 can accurately detect the fluctuation of the body without being effected by the widening of the body of the user 150 occurring due to the breathing.

<Different examples>

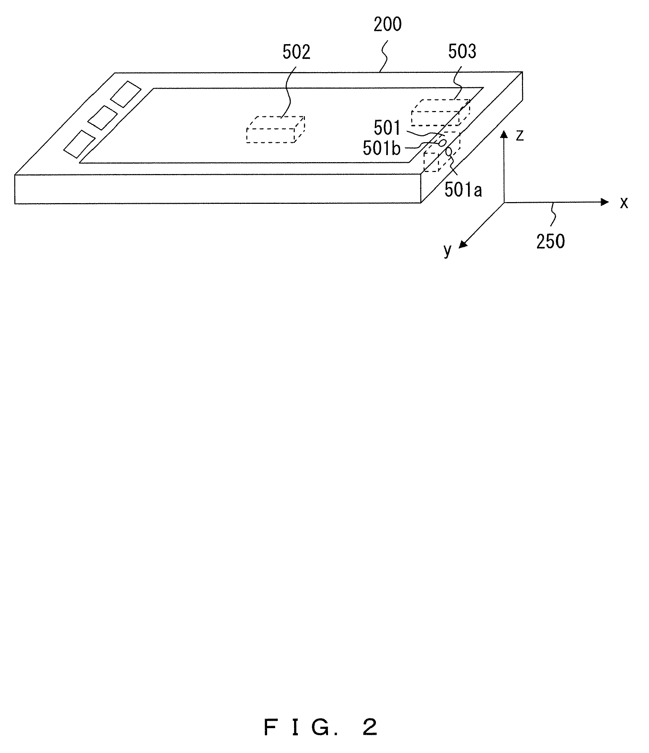

FIG. 2 illustrates an outline of a sensor included in a fluctuation detection device 200 according to a different example. The fluctuation detection device 200 illustrated in FIG. 2 is based on a case of a detection device including a fluctuation detection function as part of a mobile terminal device, however, it is not intended to limit the detection device 200 to a mobile terminal device.

The fluctuation detection device 200 may include an optical sensor 501, a geomagnetic sensor 502 and an acceleration sensor 503. However, as will be explained, the geomagnetic sensor 502 is not always necessary.

The fluctuation detection device 200 can measure a distance to a still object such as a wall or the like existing in the X axis directions of a coordinate system 250 of the fluctuation detection device 200 by emitting light from an opening portion 501a of the optical sensor 501 and receiving the reflected light. Similarly, the fluctuation detection device 200 can measure a distance to a still object such as a wall or the like existing in the Z axis directions of the coordinate system 250 of the fluctuation detection device 200 by emitting light from an opening portion 501b of the optical sensor 501 and receiving the reflected light. Hereinbelow, the coordinate system 250 of the fluctuation detection device 200 is referred to as the "first coordinate system 250".

The fluctuation detection device 200 can measure the orientations of the Z axis directions of the first coordinate system 250 in a second coordinate system 350, which will be explained later, by using the geomagnetic sensor 502. Also, the fluctuation detection device 200 can measure gravity acceleration in the Y axis directions of the coordinate system 250 by using the acceleration sensor 503.

For example, as will be described later, the acceleration sensor 503 outputs an electric signal in accordance with acceleration. In such a case, the fluctuation detection device 200 can determine gravity acceleration by extracting a DC component of a signal output from the acceleration sensor 503. For example, the fluctuation detection device 200 can determine that the Y axis direction of the coordinate system 250 and the Y axis of the second coordinate system 350, which will be explained later, are identical when acceleration to the Y axis direction of the coordinate system 250 expressed by a DC component of a signal output form the acceleration sensor 503 is the maximum. Upon this, it can further determine that the Y and Z axes of the coordinate system 250 are identical and the Y and Z axes in the second coordinate system 350 are identical when the orientation of the Z axis of the coordinate system 250 in the second coordinate system 350 is identical to the Z axis direction of the second coordinate system 350.

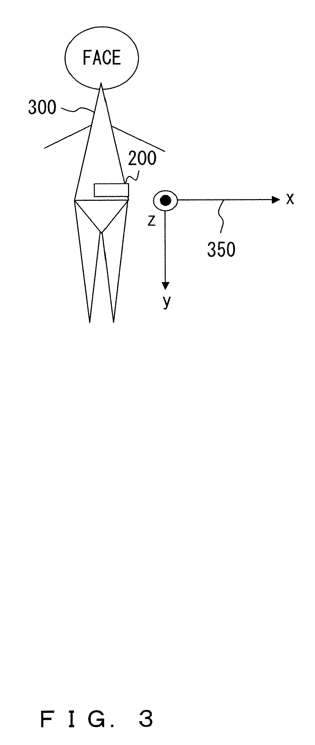

FIG. 3 illustrates an example of mounting the fluctuation detection device 200. FIG. 3 illustrates an example in a case where a user 300 wears the fluctuation detection device 200 on the waist. FIG. 3 is a side view of the user 300 standing upright on the ground. The fluctuation detection device 200 is mounted on the waist of the user 300 in such a manner that the Y axis directions of the coordinate system 250 are identical to the Y axis directions of the second coordinate system 350 of the user 300. However, it is not intended to impose a limitation that a portion on which the fluctuation detection device 200 is mounted is a waist.

Hereinbelow, the second coordinate system 350 of the user 300 illustrated in FIG. 3 is referred to as the "second coordinate system 350". The Y axis of the second coordinate system 350 has the same direction as gravity acceleration. Also, the x-z plane of the second coordinate system 350 is a plane parallel to the ground.

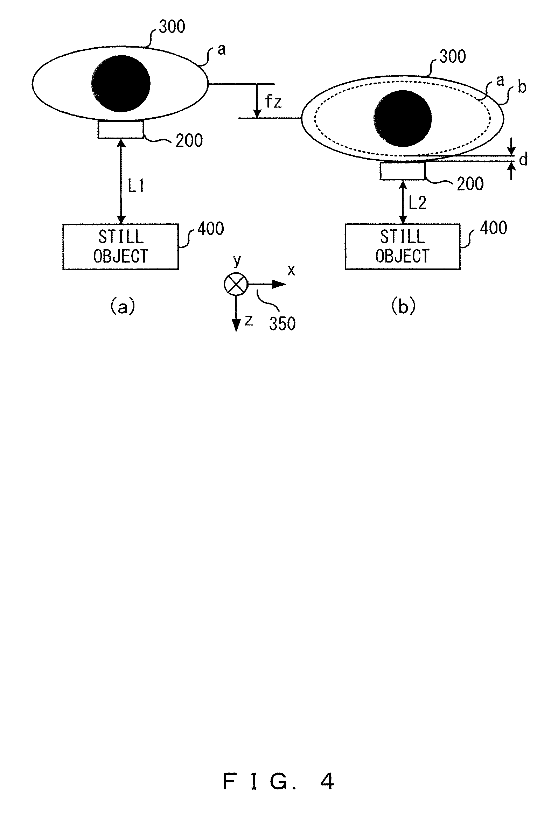

FIG. 4 illustrates an outline of fluctuation detection performed by the fluctuation detection device 200. FIG. 4 exemplifies a case where the body of the user 300 wearing the fluctuation detection device 200 fluctuated to the Z axis direction of the second coordinate system 350. FIG. 4 is a view of the user 300 seen from above.

The fluctuation detection device 200 periodically measures a distance to a still object 400 existing in the Z axis direction of the second coordinate system 350. For example, the fluctuation detection device 200 measures a distance to the still object 400 existing in the Z axis direction of the second coordinate system 350 in the state illustrated in (a) of FIG. 4. The measured distance is assumed to be L1[m].

Also, the fluctuation detection device 200 measures a distance to the still object 400 existing in the Z axis direction of the second coordinate system 350 in the state of (b) in which the body fluctuated to the Z axis direction of the second coordinate system 350 from the state of (a) of FIG. 4. The measured distance is assumed to be L2[m].

In such a case, the fluctuation detection device 200 can calculate fluctuation amount fz to the Z axis direction of the second coordinate system 350 of the user 300 from equation below (1).

$$fz = L2 - L1 \cdots \cdots (1)$$

When the user 300 has breathed in the state of (b), the body of the user 300 expands from state a to state b. In such a case, distance L2 measured by the fluctuation detection device 200 includes error d. Accordingly, the fluctuation detection device 200 according to the present example calculates fluctuation amount fz from equation (2) below when the breathing of the user 300 has been detected. Thereby, the fluctuation detection device 200 is not effected by the breathing of the user 300, making it possible to detect the fluctuation of the body of the user 300 more accurately.

$$fz=L2-L1-d\cdots\cdots(2)$$

Note that breathing includes breathing in and breathing out as a general rule. However, the present example determines the movement of expanding a body more than a prescribed level by breathing in to be breathing.

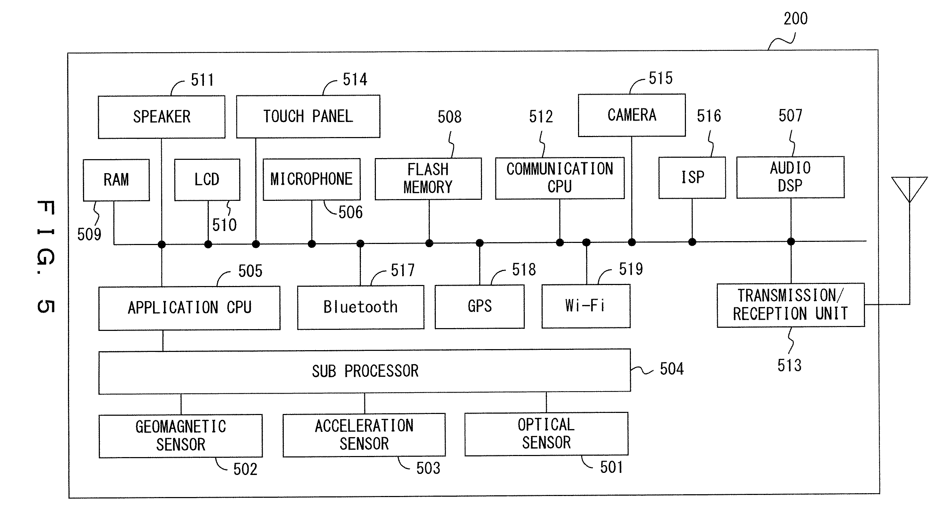

FIG. 5 illustrates an configuration example of the fluctuation detection device 200. The fluctuation detection device 200 includes an optical sensor 501, a geomagnetic sensor 520, an acceleration sensor 503, a sub processor 504, an application CPU (Central Processing Unit) 505, a microphone 506, an audio DSP 507, a flash memory 508, a RAM (Random Access Memory) 509, and an LCD (Liquid Crystal Display) 510.

The optical sensor 501 emits directional light and receives light reflected from the still object 400. The geomagnetic sensor 502 outputs an electric signal in accordance with the geomagnetism in three axis directions e.g. , the X axis direction, the Y axis direction and the Z axis direction of the first coordinate system 250. The acceleration sensor 503 outputs an electric signal in accordance with acceleration in three axis directions e.g., the X axis direction, the Y axis direction and the Z axis direction of the first coordinate system 250.

The sub processor 504 manipulates the optical sensor 501, the geomagnetic sensor 502 and the acceleration sensor 503 in accordance with an instruction from the application CPU 505. Then, the sub processor 504 converts an electric signal output from the geomagnetic sensor 502 and the acceleration sensor 503 into a digital signal, and reports it to the application CPU 505.

The application CPU 505 is a processor that executes a prescribed program. The application CPU 505 can implement the fluctuation detection device 200 according to the present example by executing a prescribed program instruction developed in the RAM 509, or the like.

The microphone 506 is a conversion device that converts audio into electric signals. The audio DSP 507 generates audio data from electric signals output from the microphone 506. Also, audio data according to the present example may include not only sound uttered by human as spoken language but also various other sounds such as breathing sound of human or the like.

The flash memory 508 is a storage device that stores a program executed by the application CPU 505 or the like and various pieces of data. The RAM 509 is a storage device that stores a program read from the flash memory 508.

The LCD 510 is a display device that displays arbitrary data. The LCD 510 may display data output from the application CPU 505 such as for example a result of detection performed by the fluctuation detection device 200 according to the present example.

Also, when the fluctuation detection device 200 is implemented by a mobile terminal device, the fluctuation detection device 200 may include a speaker 511, a communication CPU 512 and a transmission/reception unit 513.

The speaker 511 is a device that reproduces audio data transmitted from a mobile terminal device serving as a communication partner, and outputs the result.

The communication CPU 512 is a processor that executes a prescribed program and manipulates the transmission/reception unit 513 or the like so as to perform data communication. For example, the communication CPU 512 may output audio data output from the microphone 506, to the transmission/reception unit 513 so as to transmit it to a base station or the like. Also, the communication CPU 512 may output audio data received by the transmission/reception unit 513 to the speaker 511 so as to reproduce it.

The transmission/reception unit 513 converts electronic data transmitted from the communication CPU 512 into electric waves in accordance with a prescribed protocol, and outputs it to a base station or the like. The transmission/reception unit 513 converts received electric waves into electronic data, and outputs it to the communication CPU 512.

Further, the fluctuation detection device 200 may further include a touch panel 514, a camera 515, an Image Signal Processor (ISP) 516, a Bluetooth 517, a Global Positioning System (GPS) 518 and Wireless Fidelity (Wi-Fi) 519.

The touch panel 514 displays electric data output from the application CPU 505 and detects that a user has touched the screen with a finger, a pen, or the like and reports to the application CPU 505 information of the screen position of the touching. Thereby, the application CPU 505 can execute various processes in accordance with information of screen position touched by the user 300.

The camera 515 is a image pickup device that generates an electric signal from an image obtained through a lens (not illustrated) included in the camera 515 i.e., generates a image pickup signal in accordance with an instruction from the application CPU 505 etc. The camera 515 outputs generated image pickup signal to the ISP 516 or the like.

The ISP 516 is a processor that performs processes of converting image pickup data transmitted from the camera 515 into digital data i.e. , converting into image pickup data. The ISP 516 outputs the converted image pickup data to the application CPU 505 or the like. The Bluetooth 517, the GPS 518 and the Wi-Fi 519 are known techniques and explanations thereof will be omitted.

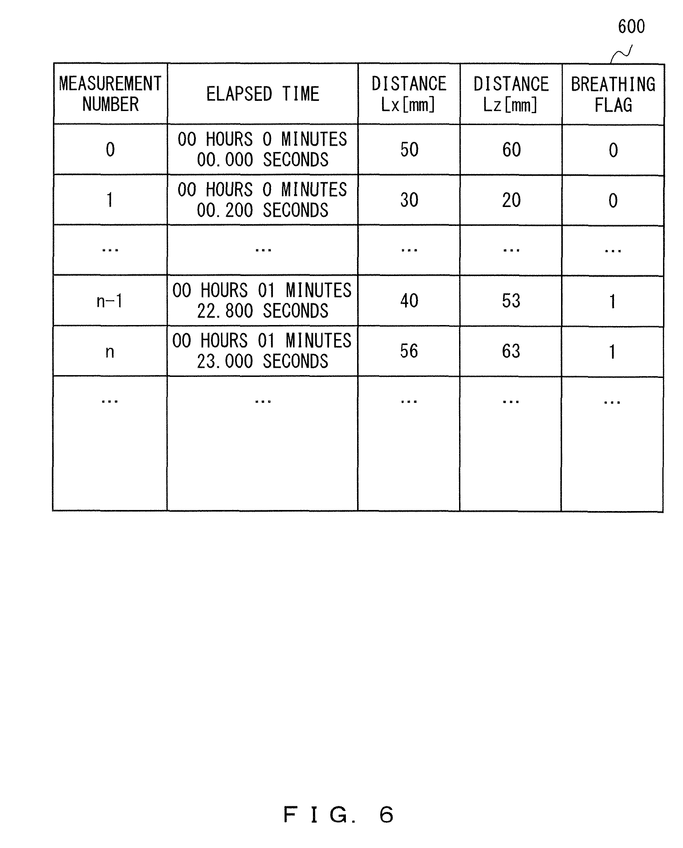

FIG. 6 illustrates an example of a shared information table 600. The shared information table 600 illustrated in FIG. 6 is information including, for each measurement number, an elapsed time, distance Lx[mm], distance Lz [mm] and a breathing flag. "n" in FIG. 6 is an integer equal to or greater than zero.

Measurement numbers are numbers that the fluctuation detection device 200 assigns to measured distances Lx and Lz sequentially starting from zero each time distance Lx and distance Lz are measured. An elapsed time is a time elapsed since the start of measurement. Distance Lx is a distance to a still object existing in the X axis direction of the first coordinate system 250. Distance Lz is a distance to a still object existing in the Z axis direction of the first coordinate system 250. A breathing flag is a flag representing whether presence or absence of breathing at a time of measuring distances Lx and Lz. When a breathing flag is "1", it represents that there was breathing. When a breathing flag is "0", it represents that there as not breathing.

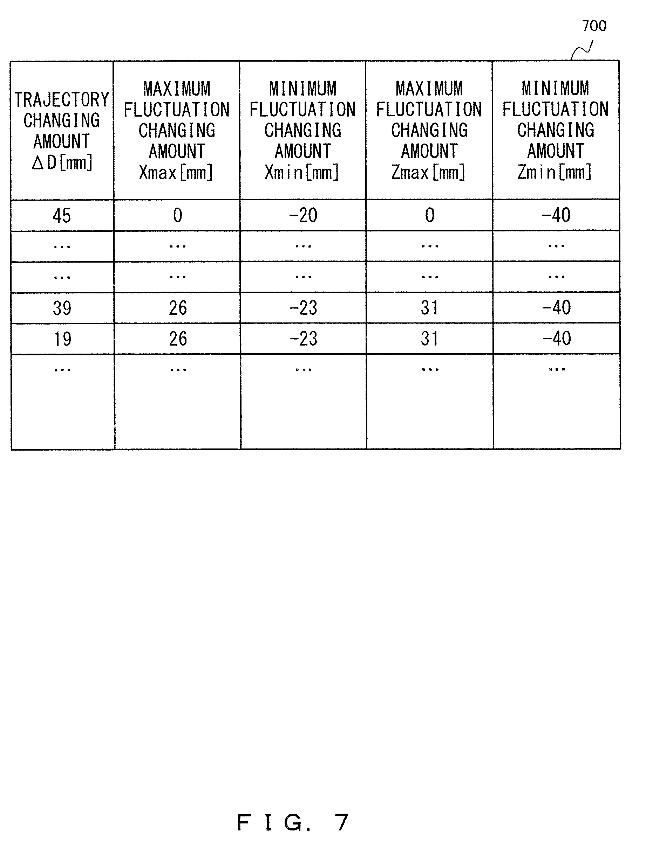

FIG. 7 illustrates an example of an information management table 700. The information management table 700 illustrated in FIG. 7 is information including trajectory changing amount $\Delta D$[mm], maximum fluctuation changing amount $X_{max}$[mm], minimum fluctuation changing amount $X_{min}$[mm], maximum fluctuation changing amount $Z_{max}$[mm] and minimum fluctuation changing amount $Z_{min}$[mm].

Trajectory changing amount $\Delta D$ is an amount of changes in a fluctuation trajectory of the user 300. Maximum fluctuation changing amount $X_{max}$ is a maximum value of a fluctuation amount in the X axis directions of the second coordinate system 350 with respect to a measurement starting position. Minimum fluctuation changing amount $X_{min}$ is a minimum value of a fluctuation amount in the X axis directions of the second coordinate system 350 with respect to a measurement starting position. Maximum fluctuation changing amount $Z_{max}$ is a maximum value of a fluctuation amount in the Z axis directions of the second coordinate system 350 with respect to a measurement starting position. Minimum fluctuation changing amount $Z_{min}$ is a minimum value of a fluctuation amount in the Z axis directions of the second coordinate system 350 with respect to a measurement starting position.

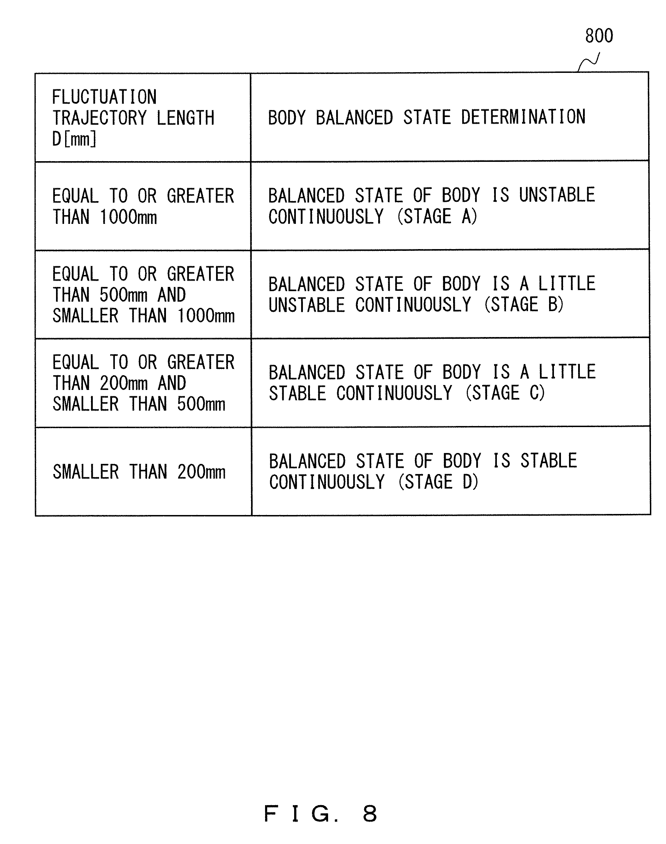

FIG. 8 illustrates an example of a first determination table 800. The first determination table 800 is information that defines the balanced state of the body of the user 300 wearing the fluctuation detection device 200, in accordance with fluctuation trajectory length D[mm].

In the example in FIG. 8, when fluctuation trajectory length D[mm] is equal to or longer than 1000[mm], it is defined that the balanced state of the body of the user 300 is unstable continuously. This state is assumed to be stage A. When fluctuation trajectory length D[mm] is equal to or longer than 500 [mm] and smaller than 1000[mm], it is defined that the balanced state of the body of the user 300 is a little unstable. This state is assumed to be stage B. Also, when fluctuation trajectory length D[mm] is equal to or longer than 200[mm] and smaller than 500[mm], it is defined that the balanced state of the body of the user 300 is a little stable. This state is assumed to be stage C. Also, when fluctuation trajectory length D [mm] is smaller than 200 [mm], it is defined that the balanced state of the body of the user 300 is continuously stable. This state is assumed to be stage D.

Also, the first determination table 800 illustrated in FIG. 8 is an example of a first determination table according to the present example and is not intended to limit the first determination table of the present example to the contents illustrated in FIG. 8.

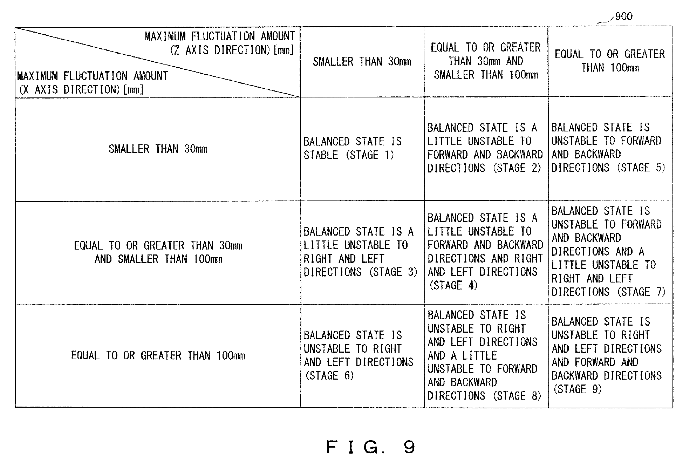

FIG. 9 illustrates an example of a second determination table 900. The second determination table 900 is information that defines the balanced state of the body of the user 300 wearing the fluctuation detection device 200, in accordance with the maximum fluctuation amount [mm] in the X axis directions of the second coordinate system 350 and the maximum fluctuation amount[mm] in the Z axis directions of the second coordinate system 350.

In the example illustrated in FIG. 9, it is defined that the balanced state of the body of the user 300 is a stable state when the maximum fluctuation amount in the X axis directions of the second coordinate system 350 is smaller than 30 [mm] and the maximum fluctuation amount in the Z axis directions of the second coordinate system 350 is smaller than 30 [mm]. This state is assumed to be stage 1.

When the maximum fluctuation amount in the X axis directions of the second coordinate system 350 is smaller than 30 [mm] and the maximum fluctuation amount in the Z axis direction of the second coordinate system 350 is equal to or greater 30 [mm] and smaller than 100 [mm], it is defined that the balanced state with respect to the forward and backward directions of the body of the user 300 is a little unstable. This state is assumed to be stage 2. Note that the forward and backward directions in the example in FIG. 9 are the forward and backward directions in a case when the user 300 directs the body front to the positive direction of the Z axis of the second coordinate system 350.

When the maximum fluctuation amount in the X axis directions of the second coordinate system 350 is equal to or greater than 30[mm] and smaller than 100[mm] and the maximum fluctuation amount in the Z axis directions of the second coordinate system 350 is smaller than 30 [mm], it is defined that the balanced state with respect to the right and left directions of the body of the user 300 is a little unstable. This stage is assumed to be stage 3. Also, the right and left directions in the example illustrated in FIG. 9 is the right and left directions in a case when the user 300 directs the body front to the positive direction of the Z axis of the second coordinate system 350.

Similarly, in FIG. 9, definition is given to a case when the maximum fluctuation amount in the X axis directions of the second coordinate system 350 is equal to or greater than 30 [mm] and smaller than 100 [mm] and the maximum fluctuation amount in the Z axis directions of the second coordinate system 350 is equal to or greater than 30[mm] and smaller than 100[mm]. In such a case, it is defined that the balanced state with respect to the forward and backward directions and the right and left directions of the body of the user 300 is a little unstable. This state is assumed to be stage 4.

Similarly, in FIG. 9, definition is given to a case when the maximum fluctuation amount in the X axis directions of the second coordinate system 350 is smaller than 30[mm] and the maximum fluctuation amount in the Z axis directions of the second coordinate system 350 is equal to or greater than 100 [mm]. In such a case, it is defined that the balanced state with respect to the forward and backward directions of the body of the user 300 is unstable. This state is assumed to be stage 5.

Similarly, in FIG. 9, definition is given to a case when the maximum fluctuation amount in the X axis directions of the second coordinate system 350 is equal to or greater than 100 [mm] and the maximum fluctuation amount in the Z axis directions of the second coordinate system 350 is smaller than 30[mm]. In such a case, it is defined that the balanced state with respect to the right and left directions of the body of the user 300 is unstable. This state is assumed to be stage 6.

Similarly, in FIG. 9, definition is given to a case when the maximum fluctuation amount in the X axis directions of the second coordinate system 350 is equal to or greater than 30 [mm] and smaller than 100 [mm] and the maximum fluctuation amount in the Z axis directions of the second coordinate system 350 is equal to or greater than 100 [mm]. In such a case, it is defined that the balanced state with respect to the forward and backward directions of the body of the user 300 is unstable and the balanced state with respect to the right and left directions of the body of the user 300 is a little unstable. This state is assumed to be stage 7.

Similarly, in FIG. 9, definition is given to a case when the maximum fluctuation amount in the X axis directions of the second coordinate system 350 is equal to or greater than 100 [mm] and the maximum fluctuation amount in the Z axis directions of the second coordinate system 350 is equal to or greater than 30 [mm] and smaller than 100 [mm]. In such a case, it is defined that the balanced state with respect to the right and left directions of the body of the user 300 is unstable and the balanced state with respect to the forward and backward directions of the body of the user 300 is a little unstable. This state is assumed to be stage 8.

Similarly, in FIG. 9, definition is given to a case when the maximum fluctuation amount in the X axis directions of the second coordinate system 350 is equal to or greater than 100 [mm] and the maximum fluctuation amount in the Z axis directions of the second coordinate system 350 is equal to or greater than 100 [mm]. In such a case, it is defined that the balanced state with respect to the right and left directions and the forward and backward directions of the body of the user 300 is unstable. This state is assumed to be stage 9.

Note that the second determination table 900 illustrated in FIG. 9 is an example of a second determination table of the present example, and is not intended to limit a second determination table of the present example to the contents illustrated in FIG. 9.

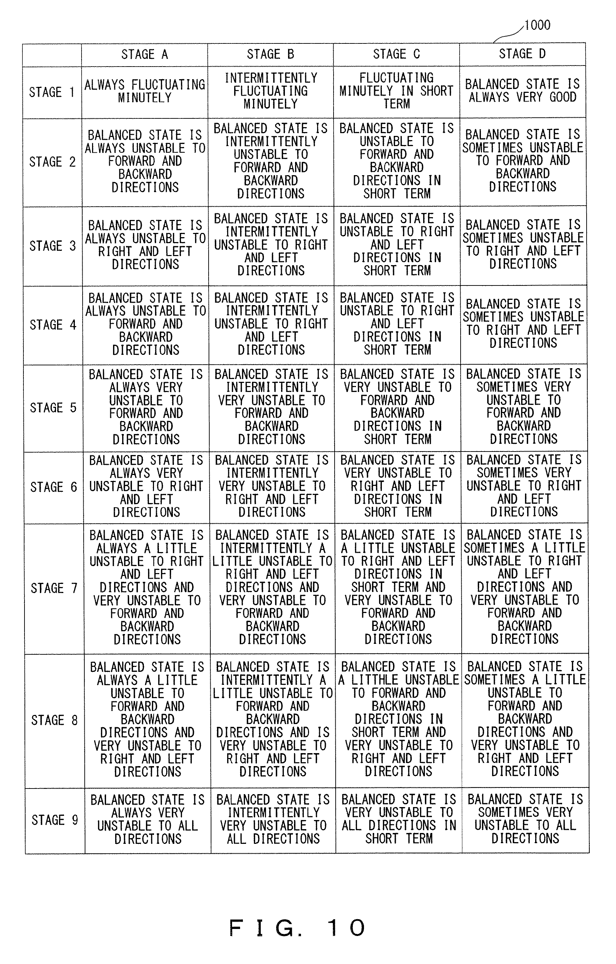

FIG. 10 illustrates an example of a total determination table 1000. The total determination table 1000 is information that defines the state of a body in accordance with a determination result (stages A through D) based on the first determination table 800 and a determination result (stages 1 through 9) based on the second determination table 900.

When, for example, the determination result based on the first determination table 800 is stage A and the determination result based on the second determination table 900 is stage 1, the fluctuation detection device 200 can determine that the body of the user 300 is always fluctuating minutely.

Note that the total determination table 1000 illustrated in FIG. 10 is an example of a total determination table of the present example, and is not intended to limit a total determination table of the present example to the contents illustrated in FIG. 10.

FIG. 11 is a flowchart illustrating processes performed by the fluctuation detection device 200. The fluctuation detection device 200 obtains values of acceleration respectively in the three axis directions of the coordinate system250 from the acceleration sensor 503 (step S1101). Then, the fluctuation detection device 200 determines whether or not the Y axis directions of the coordinate system 250 and the directions of the gravity acceleration i.e., the Y axis directions of the second coordinate system 350 are identical (NO in step S1102).

A case is assumed for example in which DC components of values of acceleration (ax, ay, az) in the X, Y and Z directions of the coordinate system 250 obtained from the acceleration sensor 503 are given from voltage values (Vax, Vay, Vaz). When Vax=Vaz=0 are satisfied, the fluctuation detection device 200 can determine that the Y axis directions of the coordinate system 250 and the directions of the gravity acceleration i.e., the Y axis directions of the second coordinate system 350 are identical.

When it is determined that the Y axis directions of the coordinate system 250 and the directions of the gravity acceleration are identical (YES in step S1102), the fluctuation detection device 200 starts the obtainment of audio data by using the microphone 506 (step S1103).

When there are no termination requests (NO in step S1104), the fluctuation detection device 200 activates threads 1 through 3 (steps S1105 through S1107). Also, when a termination request has been received (YES in step S1104), the fluctuation detection device 200 stops the obtainment of audio data that uses the microphone 506 and the emission of light that uses the optical sensor 501 (step S1108). A termination request is for example a request reported when the user 300 manipulates an input device such as a termination button included in the fluctuation detection device 200.

The fluctuation detection device 200 calculates fluctuation trajectory length D from the information management table 700 (step S1109). The fluctuation detection device 200 can calculate fluctuation trajectory length D on the basis of the total of trajectory changing amounts $\Delta D$ in the information management table 700.

Also, the fluctuation detection device 200 calculates maximum fluctuation amount Lxmax in the X axis directions of the second coordinate system 350 and maximum fluctuation amount Lzmax in the Z axis directions of the second coordinate system 350 (step S1110). For example, the fluctuation detection device 200 can calculate maximum fluctuation amount Lxmax in the X axis directions of the second coordinate system 350 from the absolute value of a difference between maximum fluctuation changing amount Xmax last added to the information management table 700 and minimum fluctuation changing amount Xmin (=|Xmax-Xmin|). Similarly, the fluctuation detection device 200 can calculate maximum fluctuation amount Lzmax in the Z axis directions of the second coordinate system 350 from the absolute value of a difference between maximum fluctuation changing amount Zmax last added to the information management table 700 and minimum fluctuation changing amount Zmin (=|Zmax-Zmin|).

Then, the fluctuation detection device 200 determines the balanced state of the state the body of the user 300 wearing the fluctuation detection device 200 on the basis of fluctuation trajectory length D calculated in step S1109 and maximum fluctuation amounts Lxmax and Lzmax calculated in step S1110 (step S1111).

The fluctuation detection device 200 can perform the determination of the balanced state of a body in the following manner.

(1) The fluctuation detection device 200 refers to the first determination table 800 and determines the body balanced state (stages A through D) in accordance with fluctuation trajectory length D calculated in step S1109.
(2) Also, the fluctuation detection device 200 refers to the second determination table 900 and determines the body balanced state (stages 1 through 9) in accordance with maximum fluctuation amounts Lxmax and Lzmax calculated in step S1110.
(3) Further, the fluctuation detection device 200 refers to the total determination table 1000 and determines the body balanced state in accordance with the body balanced state (stages A through D) determined in (1) and the body balanced state (stages 1 through 9) determined in (2).

When a determination process of a body balanced state is terminated, the fluctuation detection device 200 reports a result of determination of the body balanced state to the user 300 (step S1112). In order to report a determination result of a body balanced state to the user 300, the fluctuation detection device 200 may display a determination result in the LCD 510. Also, the fluctuation detection device 200 may report, by using an electronic mail or the like, a determination result of a body balanced state to a different mobile terminal device that is communicatable via the transmission/reception unit 513, an information processing device in a network, or the like.

When the above processes are terminated, the fluctuation detection device 200 terminates the fluctuation detection process (step S1113).

The fluctuation detection device 200 determined whether or not the Y axis directions of the coordinate system 250 and the directions of the gravity acceleration are identical by using the acceleration sensor 503 in steps S1101 and S1102. In addition to this process, the fluctuation detection device 200 can determine whether or not the Z axis directions of the coordinate system 250 and the Z axis directions of the second coordinate system 350 are identical. In other words, in steps S1101 and S1102, the fluctuation detection device 200 determines whether or not the Y axis directions of the coordinate system 250 and the directions of the gravity acceleration are identical and whether or not the Z axis directions of the coordinate system 250 and the Z axis directions of the second coordinate system 350 are identical in step S1101 and S1102. When the orientation of the Z axis direction of the second coordinate system 350 is south and the orientation of the Z axis direction of the coordinate system 250 obtained by the geomagnetic sensor 502 is south, the fluctuation detection device 200 can determine that the Z axis direction of the coordinate system 250 and the Z axis direction of the second coordinate system 350 are identical.

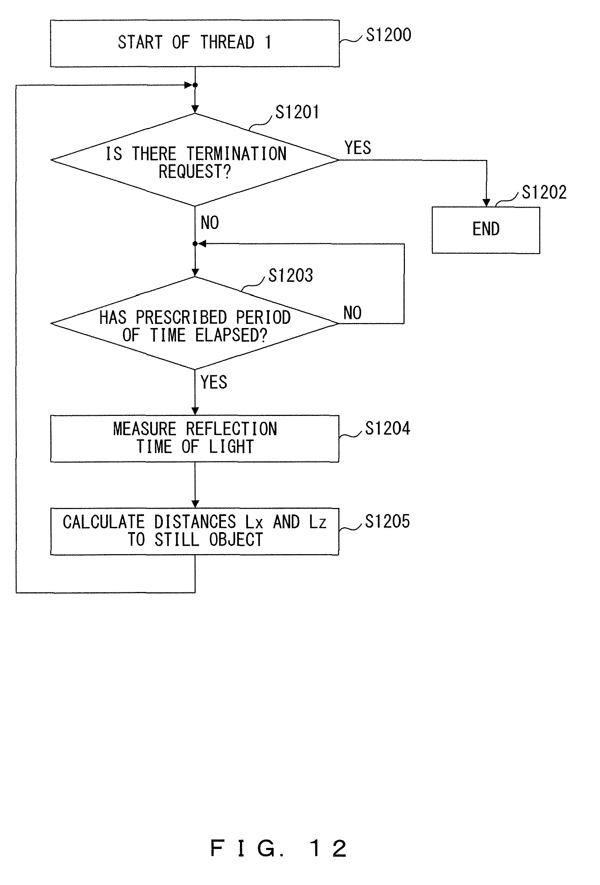

FIG. 12 is a flowchart illustrating a process of thread 1 executed by the fluctuation detection device 200. When a termination request has been received (YES in step S1201), the fluctuation detection device 200 terminates the process of thread 1 (step S1202). When there are no termination requests (NO in step S1201), the fluctuation detection device 200 makes the process proceed to step S1203. In such a case, the fluctuation detection device 200 waits for a prescribed period of time e.g., 0.2 seconds (NO in step S1203). This is for securing a period of time for the obtainment of audio data that started in step S1103. However, it is not intended to limit the period of time of waiting to 0.2 seconds.

When a prescribed period of time has elapsed (YES in step S1203), the fluctuation detection device 200 makes the optical sensor 501 emit light to the X axis directions and the Y axis directions of the coordinate system 250 and receive the reflection light thereof. Then, the fluctuation detection device 200 measures reflection time tx (step S1204) between the emission of light to the X axis directions of the coordinate system 250 and the reception of the reflection light (step S1204). Similarly, the fluctuation detection device 200 measures reflection time tz (step S1204) between the emission of light to the Z axis directions of the coordinate system 250 and the reception of the reflection light.

The fluctuation detection device 200 calculates distance Lx to a still object existing in the X axis directions of the coordinate system 250 on the basis of reflection time tx measured in step S1204 (step S1205). Similarly, the fluctuation detection device 200 calculates distance Lz to a still object existing in the Z axis directions of the coordinate system 250 on the basis of reflection time tz measured in step S1204 (step S1205). Then, the fluctuation detection device 200 adds the calculated distances Lx and Lz to the shared information table 600. In such a case, the fluctuation detection device 200 sets, in the shared information table 600, measurement numbers for the added distances Lx and Lz and the periods of time that elapsed since the start of the execution of thread 1 to the measurement of the added distances Lx and Lz.

When the above processes are completed, the fluctuation detection device 200 makes the process proceed to step S1201.

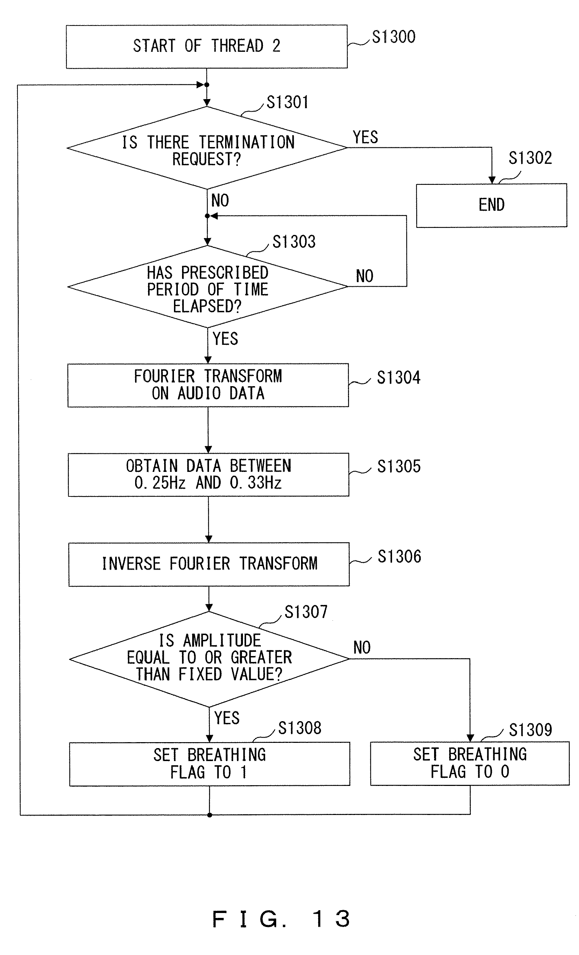

FIG. 13 is a flowchart illustrating processes of thread 2 executed by the fluctuation detection device 200. When a termination request is received (YES in step S1301), the fluctuation detection device 200 terminates processes in thread 2 (step S1302). When there are no termination requests (NO in step S1301), the fluctuation detection device 200 makes the process proceed to step S1303. In such a case, the fluctuation detection device 200 waits for a prescribed period of time e.g., 0.2 seconds (NO in step S1303). This is for securing a period of time for the obtainment of audio data that started in step S1103. However, it is not intended to limit the period of time of waiting to 0.2 seconds.

When a prescribed period of time has elapsed (YES in step S1303), the fluctuation detection device 200 performs Fourier transform on audio data of the prescribed period of time (step S1304). Then, the fluctuation detection device 200 obtains a amplitude value of a signal in a frequency band occurring during breathing from the audio data that is Fourier transformed (step S1305). In the present example, audio data in the frequency band from 0.25Hz to 0.33Hz is obtained. However, it is not intended to limit a frequency band of obtained audio data to a band from 0.25Hz to 0.33Hz. The fluctuation detection device 200 performs inverse Fourier transform on the audio data obtained in step S1305 (step S1306).

When the amplitude value of the audio data obtained in the process in step S1306 is equal to or greater than a fixed value (YES in step S1307), the fluctuation detection device 200 determines that the user is breathing. In such a case, the fluctuation detection device 200 sets, to "1", the breathing flag corresponding to the measurement number, in the shared information table 600, that was last added in step S1205 (step S1308). Also, when the amplitude value of the audio data obtained in the process in step S1306 is smaller than a fixed value (NO in step S1307), the fluctuation detection device 200 determines that the user is not breathing. In such a case, the fluctuation detection device 200 sets, to "0", the breathing flag corresponding to the measurement number, in the shared information table 600, that was added in step S1205 (step S1309).

When the process in step S1308 or step S1309 is completed, the fluctuation detection device 200 makes the process proceed to step S1301.

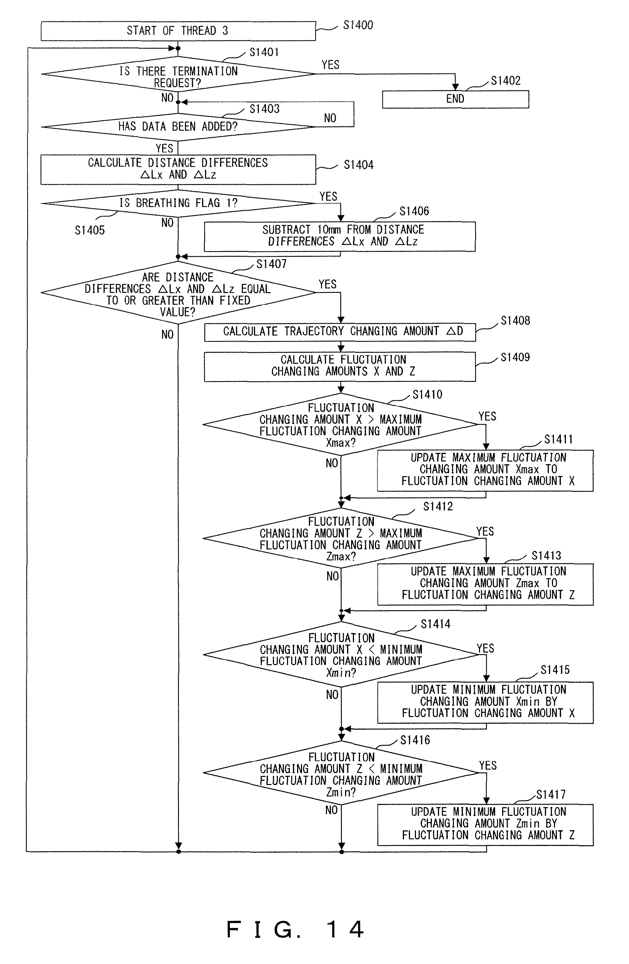

FIG. 14 is a flowchart illustrating processes of thread 3 executed by the fluctuation detection device 200. When a termination request is received (YES in step S1401), the fluctuation detection device 200 terminates processes in thread 3 (step S1402). When there are no termination requests (NO in step S1401), the fluctuation detection device 200 makes the process proceed to step S1403.

The fluctuation detection device 200 waits for new pieces of shared information i.e., distances Lx and Lz and a breathing flag to be added to the shared information table 600 (NO in step S1403). When new pieces of shared information have been added to the shared information table 600 (YES in step S1403), the fluctuation detection device 200 makes the process proceed to step S1404. In such a case, the fluctuation detection device 200 calculates distance difference ΔLx with respect to the X axis directions of the second coordinate system 350 and distance difference ΔLz with respect to Z axis directions of the second coordinate system 350 (step S1404). This distance difference ΔLx is a fluctuation amount of the body of the user 300 in the X axis directions of the second coordinate system 350 during a fixed period of time. Similarly, distance difference ΔLz is a fluctuation amount of the body of the user 300 in the Z axis directions of the second coordinate system 350 during a fixed period of time.

When for example it is assumed that the measurement number of a piece of shared information newly added to the shared information table 600 is n, distances Lx and Lz of measurement number n are distances Lxn and Lzn, respectively, the fluctuation detection device 200 can calculate distance difference ΔLx in the X axis directions of the second coordinate system 350 by the following equation.

$$\Delta Lx = |Lxn - Lx(n-1)| \cdots (3)$$

Similarly, the fluctuation detection device 200 can calculate distance difference ΔLz in the Y axis directions of the second coordinate system 350 by the following equation.

$$\Delta Lz = |Lzn - Lz(n-1)| \cdots (4)$$

When the breathing flag included in the piece of shared information newly added to the shared information table 600 is one (YES in step S1405), the fluctuation detection device 200 respectively subtracts 10 [mm] from distance differences $\Delta Lz$ and $\Delta Lz$ calculated in step S1404 (step S1406). However, it is not intended to limit the value subtracted from distance differences $\Delta Lx$ and $Lz$ to 10[mm]. It is also possible to use for example a value that dynamically varies in accordance with the amplitude value of audio data calculated in step S1306 instead of a fixed value such as 10 [mm] as a value to be subtracted from distance differences $\Delta Lx$ and $\Delta Lz$. It is also possible that distance differences $\Delta Lx$ and $Lz$ are values different from each other.

When the process in step S1406 is terminated, the fluctuation detection device 200 makes the process proceed to step S1407. Also, when the breathing flag included in the piece of shared information newly added to the shared information table 600 is zero (NO in step S1405), the fluctuation detection device 200 makes process proceed to step S1407.

When the distance differences $\Delta Lx$ and $\Delta Lz$ calculated in step S1404 are smaller than a fixed value (NO in step S1407), the fluctuation detection device 200 makes the process proceed to step S1401. This process is for eliminating a measurement error of distances $Lx$ and $Lz$. Accordingly, a fixed value used for the comparison between distance differences $\Delta Lx$ and $\Delta Lz$ may be determined by the measurement performance of the fluctuation detection device 200 i.e., the measurement error caused in a measurement of distances $Lx$ and $Lz$.

When distance differences $\Delta Lx$ and $\Delta Lz$ calculated in step S1404 are equal to or greater than a fixed value (YES in step S1407), the fluctuation detection device 200 makes the process proceed to step S1408. In such a case, the fluctuation detection device 200 calculates the variation amount of trajectory of the fluctuation of the body of the user 300 i.e. , trajectory changing amount $\Delta D$ (step S1408). Then, the fluctuation detection device 200 adds calculated trajectory changing amount $\Delta D$ to the information management table 700. The fluctuation detection device 200 can calculate trajectory changing amount $\Delta D$ by the equation below.

$$\Delta D = \sqrt{(\Delta Lx \times \Delta Lx + \Delta Lz \times \Delta Lz)} \cdots (5)$$

Also, the fluctuation detection device 200 calculates fluctuation changing amounts $X$ and $Z$ (step S1409). When for example distances $Lx$ and $Lz$ of measurement number 0 in the shared information table 600 are assumed to be distances $Lx0$ and $Lz0$, the fluctuation detection device 200 can calculate fluctuation changing amounts $X$ and $Z$ by the following equations.

$$X = Lxn - Lx0 \cdots (6)$$

$$Z = Lzn - Lz0 \cdots (7)$$

The fluctuation detection device 200 obtains, from the information management table 700, maximum fluctuation changing amount $Xmax$ that was last added. Then, the fluctuation detection device 200 compares obtained maximum fluctuation changing amount $Xmax$ and fluctuation changing amount $X$ calculated in step S1409.

When changing amount $X$ calculated in step S1409 is greater than maximum fluctuation changing amount $Xmax$ (YES in step S1410), the fluctuation detection device 200 makes the process proceed to step S1411. In such a case, the fluctuation detection device 200 adds fluctuation changing amount $X$ calculated in step S1409 to maximum fluctuation changing amount $Xmax$ in the information management table 700 (step S1411). Then, the fluctuation detection device 200 makes the process proceed to step S1412.

When fluctuation changing amount $X$ calculated in step S1409 is equal to or smaller than maximum fluctuation changing amount $Xmax$ (NO in step S1410), the fluctuation detection device 200 adds maximum fluctuation changing amount $Xmax$ that is the same as in the previous time to maximum fluctuation changing amount $Xmax$ in the information management table 700. Then, the fluctuation detection device 200 makes the process proceed to step S1412.

The fluctuation detection device 200 obtains maximum fluctuation changing amount $Zmax$ that was last added from the information management table 700. Then, the fluctuation detection device 200 compares obtained maximum fluctuation changing amount $Zmax$ and fluctuation changing amount $Z$ calculated in step S1409.

When fluctuation changing amount $Z$ calculated in step S1409 is greater than maximum fluctuation changing amount $Zmax$ (YES in step S1412), the fluctuation detection device 200 makes the process proceed to step S1413. In such a case, the fluctuation detection device 200 adds fluctuation changing amount $Z$ calculated in step S1409 to maximum fluctuation changing amount $Zmax$ in the information management table 700 (step S1413). Then, the fluctuation detection device 200 makes the process proceed to step S1414.

Also, when fluctuation changing amount $Z$ calculated in step S1409 is equal to or smaller than maximum fluctuation changing amount $Zmax$ (NO in step S1412) , the fluctuation detection device 200 adds maximum fluctuation changing amount $Zmax$ that is the same as in the previous time to maximum fluctuation changing amount $Xmax$ in the information management table 700. Then, the fluctuation detection device 200 makes the process proceed to step S1414.

The fluctuation detection device 200 obtains minimum fluctuation changing amount $Xmin$ that was last added from the information management table 700. Then, the fluctuation detection device 200 compares obtained minimum fluctuation changing amount Xmin and fluctuation changing amount X calculated in step S1409.

When fluctuation changing amount X calculated in step S1409 is smaller than minimum fluctuation changing amount Xmin (YES in step S1414), the fluctuation detection device 200 makes the process proceed to step S1415. In such a case, the fluctuation detection device 200 adds fluctuation changing amount X calculated in step S1409 to minimum fluctuation changing amount Xmin in the information management table 700 (step S1415). Then, the fluctuation detection device 200 makes the process proceed to step S1416.

Also, when fluctuation changing amount X calculated in step S1409 is equal to or greater than minimum fluctuation changing amount Xmin (NO in step S1414), the fluctuation detection device 200 adds minimum fluctuation changing amount Xmin that is the same as in the previous time to minimum fluctuation changing amount Xmin in the information management table 700. Then, the fluctuation detection device 200 makes the process proceed to step S1416.

The fluctuation detection device 200 obtains minimum fluctuation changing amount Zmin that was last added from the information management table 700. Then, the fluctuation detection device 200 compares obtained minimum fluctuation changing amount Zmin and fluctuation changing amount Z calculated in step S1409.

When fluctuation changing amount Z calculated in step S1409 is smaller than minimum fluctuation changing amount Zmin (YES in step S1416), the fluctuation detection device 200 makes the process proceed to step S1417. In such a case, the fluctuation detection device 200 adds fluctuation changing amount Z calculated in step S1409 to minimum fluctuation changing amount Zmin in the information management table 700 (step S1417). Then, the fluctuation detection device 200 makes the process proceed to step S1401.

Also, when fluctuation changing amount Z calculated in step S1409 is equal to or smaller than minimum fluctuation changing amount Zmin (NO in step S1416), the fluctuation detection device 200 adds minimum fluctuation changing amount Zmin that is the same as in the previous time to minimum fluctuation changing amount Zmin in the information management table 700. Then, the fluctuation detection device 200 makes the process proceed to step S1401.

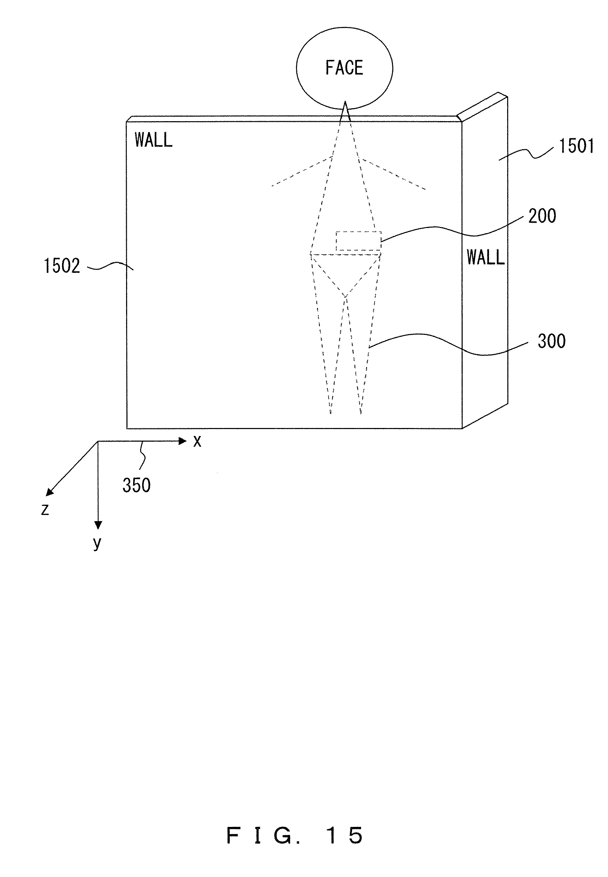

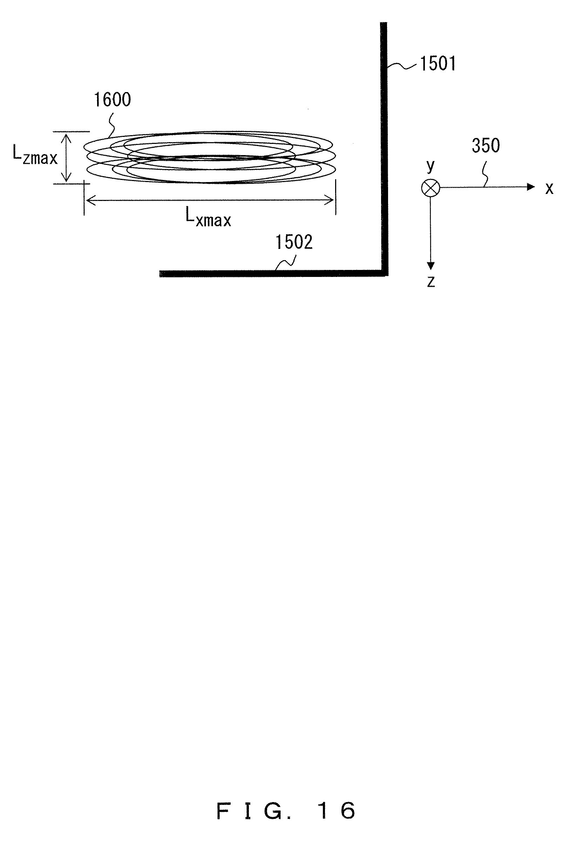

FIG. 15 and FIG. 16 illustrate an example of detecting fluctuation of the body of the user 300 by using the fluctuation detection device 200. FIG. 15 is a side view of the body for a case where the user 300 wears the fluctuation detection device 200 on his/ her waist. It is now assumed that there are walls 1501 and 1502 in the X axis direction and the Z axis direction of the second coordinate system 350, respectively and fluctuation 1600 has occurred in the body of the user 300 as illustrated in FIG. 16, which is a top view of FIG. 15. Note that the user 300 is omitted for the sake of easier understanding although FIG. 16 is a top view of FIG. 15.

In such a case, the fluctuation detection device 200 calculates, as explained in FIG. 11 through FIG. 14, distance difference ΔLx by measuring distance Lx to the wall 1501 existing in the X axis direction of the second coordinate system 350. Similarly, the fluctuation detection device 200 calculates distance difference ΔLz by measuring distance Lz to the wall 1502 existing in the Z axis direction of the second coordinate system 350. The fluctuation detection device 200 calculates trajectory changing amounts ΔD of fluctuation of the body of the user 300 on the X-Z plane of the second coordinate system 350 from the calculated distance differences ΔLx and ΔLz. It is possible to calculate fluctuation trajectory length D of the fluctuation 1600 of the body of the user 300 from the sum of the trajectory changing amounts ΔD.

Also, the fluctuation detection device 200 calculates maximum fluctuation changing amounts Xmax and Zmax and minimum fluctuation changing amounts Xmin and Zmin from measured distances Lx and Lz. The fluctuation detection device 200 calculates maximum fluctuation amounts Lxmax and Lzmax from the calculated maximum fluctuation changing amounts Xmax and Zmax and minimum fluctuation changing amounts Xmin and Zmin.

Then, the fluctuation detection device 200 determines the balanced state of the body of the user 300 from fluctuation trajectory length D, maximum fluctuation amounts Lxmax and Lzmax, the first determination table 800, the second determination table 900 and the total determination table 1000.

In the above explanations, the X axis direction can be used as an example of a first direction. Distance Lx can be used as an example of a first distance, which is a distance to a still object existing in the first direction. The Z axis direction can be used as an example of a second direction. Distance Lz can be used as an example of a second distance, which is a distance to a still object existing in the second direction. Distance difference ΔLx can be used as an example of a first fluctuation amount, which is a changing amount of the first distance. Distance difference ΔLz can be used as an example of a second fluctuation amount, which is a changing amount of the second distance. Also, a determination result (stages A through D) based on the first determination table 800 can be used as an example of first stability. Also, a determination result (stages 1 through 9) based on the second determination table 900 can be used as an example of second stability. Also, a determination result based on the total determination table 1000 can be used as an example of stability of a balances state of the user 300. Also, the measurement unit, the calculation unit and the removal unit can be implemented by the application CPU 505 executing a prescribed program stored in the RAM 509 or the like.

As explained in the above, when the fluctuation detection device 200 has detected breathing of the user 300, the fluctuation detection device 200 uses a value resulting from subtracting the extent of the widening caused by the breathing of the user 300 i.e., 10 [mm] in the present example, from distance differences ΔLx and ΔLz. Thereby, the fluctuation detection device 200 can remove, from distance differences ΔLx and ΔLz, a measurement error included in the measurement of distances Lx and Lz i.e. , the extent of the widening of the body caused by the breathing of the user 300. As a result of this, the fluctuation detection device 200 can detect fluctuation of the body accurately without being effected by the enlargement of the body caused by breathing of the user 300 wearing the fluctuation detection device 200.

Also, the fluctuation detection device 200 detects fluctuation of the body of the user 300 on the basis of changing amounts of distances Lx and Lz, measured by using the optical sensor 501, to still objects existing in the X axis direction and the Y axis direction of the first coordinate system 250. Accordingly, it is easy to measure minute fluctuation of the body that falls below the resolution of sensors such as an acceleration sensor, a gyro sensor or the like. Also, because the optical sensor 501 is used for detecting fluctuation, it is possible to realize detection of fluctuation of a body by a simple system.

Also, the fluctuation detection device 200 calculates fluctuation trajectory length D and maximum fluctuation amounts Lxmax and Lzmax. The fluctuation detection device 200 can determine a body balanced state in accordance with calculated fluctuation trajectory length D and maximum fluctuation amounts Lxmax and Lzmax by using the first determination table 800, the second determination table 900 and the total determination table 1000.

The fluctuation detection device 200 determines whether or not the Y axis direction of the coordinate system 250 and the direction of the gravity acceleration are identical by using the acceleration sensor 503 in steps S1101 and S1102. Accordingly, the fluctuation detection device 200 starts the fluctuation detection process when the user 300 wearing the fluctuation detection device 200 is at a particular posture. Thereby, the fluctuation detection device 200 can always execute the fluctuation detection process under a particular condition. As a result of this, the fluctuation detection device 200 can improve the accuracy of fluctuation detection.

Explanation of symbols

| 100 | FLUCTUATION DETECTION DEVICE |
|---|---|
| 110 | MEASUREMENT UNIT |
| 120 | CALCULATION UNIT |
| 130 | REMOVAL UNIT |
| 150 | USER |
| 160 | STILL OBJECT |
| 170 | STILL OBJECT |

**Claims**

1. A fluctuation detection device configured to detect fluctuation of a body of a user, the fluctuation detection device comprising:

   a measurement unit (110) configured to measure a first distance that is a distance to a still object existing in a first direction and a second distance that is a distance to a still object existing in a second direction different from the first direction;
   a calculation unit (120) configured to calculate a first fluctuation amount that is a changing amount of the first distance and a second fluctuation amount that is a changing amount of the second distance; and
   a removal unit (130) including an obtainment unit (507) configured to obtain sound data of breathing of the user and a determination unit (505) configured to determine that the user is breathing when an amplitude value of a particular frequency band is equal to or greater than a fixed value among frequency bands obtained by performing Fourier transform on the sound data, wherein the removal unit is
   configured to remove a changing amount of the first distance caused by breathing of the user from the first fluctuation amount and to remove a changing amount of the second distance caused by the breathing from the second fluctuation amount, when breathing of the user is detected.

2. The fluctuation detection device according to claim 1, further comprising
   a detection unit (502,503) configured to detect a direction of a fluctuation detection device worn by the user, wherein
   when the direction is identical to a particular direction, the measurement unit(110) is configured to start measurement of the first distance and the second distance.

3. The fluctuation detection device according to claim 1, further comprising:

   a calculation unit (120) configured to calculate a length of trajectory of fluctuation of the user from the first fluctuation amount and the second fluctuation amount; and
   a determination unit (505) configured to determine a body state of the user on the basis of a length of the trajectory, a maximum value of fluctuation to the first direction and a maximum value of fluctuation to the second direction.

4. The fluctuation detection device according to claim 3, wherein
the determination unit (505) is configured to determine stability of a balanced state of the user from first stability related to a balanced state of the user in accordance with a length of the trajectory and second stability related to a balanced state of the user in accordance with a maximum value of fluctuation to the first direction and a maximum value of fluctuation to the second direction.

5. The fluctuation detection device according to claim 1, wherein
the removal unit (130) is configured to subtract a prescribed value from the first fluctuation amount and the second fluctuation amount when breathing of the user is detected during the measurement.

6. The fluctuation detection device according to claim 1, wherein
the removal unit (130) is configured to subtract a value in accordance with an amplitude value of the particular frequency band from the first fluctuation amount and the second fluctuation amount when breathing of the user is detected during the measurement.

7. A fluctuation detection method that detects fluctuation of a body of a user, the fluctuation detection method comprising:

measuring a first distance that is a distance to a still object existing in a first direction and a second distance that is a distance to a still object existing in a second direction different from the first direction (S1205);
calculating a first fluctuation amount that is a changing amount of the first distance and a second fluctuation amount that is a changing amount of the second distance (S1404);
detecting breathing of the user (S1307); and removing a changing amount of the first distance caused by the breathing from the first fluctuation amount and removing a changing amount of the second distance caused by the breathing from the second fluctuation amount when breathing of the user is detected during the measurement (S1406), when an amplitude value of a particular frequency band from among frequency bands obtained by performing Fourier transform on sound data of the breathing of the user is equal to or greater than a fixed value.

8. A program, which when executed by a fluctuation detection device, causes the fluctuation detection device to perform the method of claim 7.

**Patentansprüche**

1. Eine Fluktuationsdetektionsvorrichtung, ausgebildet zum Detektieren einer Fluktuation eines Körpers eines Anwenders, wobei die Fluktuationsdetektionsvorrichtung umfasst:

eine Messeinheit (110), ausgebildet zum Messen eines ersten Abstands, welcher ein Abstand zu einem unbewegten Objekt ist, welches sich in einer ersten Richtung befindet, und eines zweiten Abstands, welcher ein Abstand zu einem unbewegten Objekt ist, welches sich in einer zweiten Richtung unterschiedlich zu der ersten Richtung befindet;
eine Berechnungseinheit (120), ausgebildet zum Berechnen eines ersten Fluktuationsbetrags, welcher ein Änderungsbetrag des ersten Abstands ist, und eines zweiten Fluktuationsbetrags, welcher ein Änderungsbetrag des zweiten Abstands ist; und
eine Entfernungseinheit (130), umfassend eine Erfassungseinheit (507), ausgebildet zum Erfassen von Klangdaten einer Atmung des Anwenders, und eine Bestimmungseinheit (505), ausgebildet zum Bestimmen, dass der Anwender atmet, wenn ein Amplitudenwert eines bestimmten Frequenzbands gleich oder größer als ein fester Wert ist, aus Frequenzbändern, welche durch Ausführen einer Fourier-Transformation an den Klangdaten erhalten werden, wobei die Entfernungseinheit ausgebildet ist zum Entfernen eines Änderungsbetrags des ersten Abstands, welcher durch ein Atmen des Anwenders verursacht wird, von dem ersten Fluktuationsbetrag und zum Entfernen eines Änderungsbetrags des zweiten Abstands, welcher durch das Atmen verursacht wird, von dem zweiten Fluktuationsbetrag, wenn ein Atmen des Anwenders detektiert ist.

2. Fluktuationsdetektionsvorrichtung gemäß Anspruch 1, weiter umfassend
eine Detektionseinheit (502, 503), ausgebildet zum Detektieren einer Richtung einer von dem Anwender getragenen Fluktuationsdetektionsvorrichtung, wobei,
wenn die Richtung identisch zu einer bestimmten Richtung ist, die Messeinheit (110) ausgebildet ist, eine Messung des ersten Abstands und des zweiten Abstands zu starten.

3. Fluktuationsdetektionsvorrichtung gemäß Anspruch 1, weiter umfassend:

eine Berechnungseinheit (120), ausgebildet zum Berechnen einer Pfadlänge einer Fluktuation des Anwenders aus dem ersten Fluktuationsbetrag und dem zweiten Fluktuationsbetrag; und

eine Bestimmungseinheit (505), ausgebildet zum Bestimmen eines Körperzustands des Anwenders auf der Basis einer Länge des Verfahrens, eines Maximalwerts einer Fluktuation in der ersten Richtung und eines Maximalwert einer Fluktuation in der zweiten Richtung.

4. Fluktuationsdetektionsvorrichtung gemäß Anspruch 3, wobei
die Bestimmungseinheit (505) ausgebildet ist zum Bestimmen einer Stabilität eines ausgeglichenen Zustands des Anwenders aus einer einen ausgeglichenen Zustand des Anwenders betreffenden ersten Stabilität entsprechend einer Länge des Pfades und einer einem ausgeglichenen Zustand des Anwenders betreffenden zweiten Stabilität entsprechend einem Maximalwert einer Fluktuation in der ersten Richtung und einem Maximalwert einer Fluktuation in der zweiten Richtung.

5. Fluktuationsdetektionsvorrichtung gemäß Anspruch 1, wobei
die Entfernungseinheit (130) ausgebildet ist zum Subtrahieren eines vorgegebenen Werts von dem ersten Fluktuationsbetrag und dem zweiten Fluktuationsbetrag, wenn ein Atmen des Anwenders während der Messung detektiert ist.

6. Fluktuationsdetektionsvorrichtung gemäß Anspruch 1, wobei
die Entfernungseinheit (130) ausgebildet ist zum Subtrahieren eines Werts entsprechend eines Amplitudenwerts des bestimmten Frequenzbands von dem ersten Fluktuationsbetrag und dem zweiten Fluktuationsbetrag, wenn ein Atmen des Anwenders während der Messung detektiert ist.

7. Ein Fluktuationsdetektionsverfahren, welches eine Fluktuation eines Körpers eines Anwenders detektiert, wobei das Fluktuationsdetektionsverfahren umfasst:

Messen eines ersten Abstands, welcher ein Abstand zu einem unbewegten Objekt ist, welches sich in einer ersten Richtung befindet, und eines zweiten Abstands, welcher ein Abstand zu einem unbewegten Objekt ist, welches sich in einer zweiten Richtung unterschiedlich zu der ersten Richtung befindet (S1205);
Berechnen eines ersten Fluktuationsbetrags, welcher ein Änderungsbetrag des ersten Abstands ist, und eines zweiten Fluktuationsbetrags, welcher ein Änderungsbetrag des zweiten Abstands ist (S1404);
Detektieren eines Atmens des Anwenders (S1307); und

Entfernen eines Änderungsbetrags des ersten Abstands, welcher durch das Atmen verursacht wird, von dem ersten Fluktuationsbetrag und Entfernen eines Änderungsbetrags des zweiten Abstands, welcher durch das Atmen verursacht wird, von dem zweiten Fluktuationsbetrag, wenn ein Atmen des Anwenders während der Messung detektiert wird (S1406), wenn ein Amplitudenwert eines bestimmten Frequenzbands aus Frequenzbändern, welche durch Ausführen einer Fourier-Transformation an Klangdaten des Atmens des Anwenders erhalten sind, gleich oder größer als ein fester Wert ist.

8. Ein Programm, welches, wenn dieses durch eine Fluktuationsdetektionsvorrichtung ausgeführt wird, die Fluktuationsdetektionsvorrichtung zum Ausführen des Verfahrens gemäß Anspruch 7 veranlasst.

**Revendications**

1. Dispositif de détection de fluctuation configuré pour détecter la fluctuation d'un corps d'un utilisateur, le dispositif de détection de fluctuation comprenant :

une unité de mesure (110) configurée pour mesurer une première distance qui est une distance par rapport à un objet immobile existant dans une première direction et une seconde distance qui est une distance par rapport à un objet immobile existant dans une seconde direction différente de la première direction ;
une unité de calcul (120) configurée pour calculer une première quantité de fluctuation qui est une quantité changeante de la première distance et une seconde quantité de fluctuation qui est une quantité changeante de la seconde distance ; et
une unité de retrait (130) comprenant une unité d'obtention (507) configurée pour obtenir des données sonores de respiration de l'utilisateur et une unité de détermination (505) configurée pour déterminer que l'utilisateur respire lorsqu'une valeur d'amplitude d'une bande de fréquences particulière est égale ou supérieure à une valeur définie parmi des bandes de fréquences obtenues en exécutant la transformée de Fourier sur les données sonores, où l'unité de retrait est configurée pour retirer une quantité changeante de la première distance provoquée par la respiration de l'utilisateur à partir de la première quantité de fluctuation et pour retirer une quantité changeante de la seconde distance provoquée par la respiration à partir de la seconde quantité de fluctuation, lorsque la respiration de l'utilisateur est détectée.

**2.** Dispositif de détection de fluctuation selon la revendication 1, comprenant en outre :

une unité de détection (502, 503) configurée pour détecter une direction d'un dispositif de détection de fluctuation porté par l'utilisateur, où lorsque la direction est identique à une direction particulière, l'unité de mesure (110) est configurée pour démarrer la mesure de la première distance et de la seconde distante.

**3.** Dispositif de détection de fluctuation selon la revendication 1, comprenant en outre :

une unité de calcul (120) configurée pour calculer une longueur de trajectoire de fluctuation de l'utilisateur à partir de la première quantité de fluctuation et de la seconde quantité de fluctuation ; et

une unité de détermination (505) configurée pour déterminer un état physique de l'utilisateur sur la base d'une longueur de la trajectoire, d'une valeur maximale de fluctuation vers la première direction et d'une valeur maximale de fluctuation vers la seconde direction.

**4.** Dispositif de détection de fluctuation selon la revendication 3, dans lequel

l'unité de détermination (505) est configurée pour déterminer une stabilité d'un état équilibré de l'utilisateur à partir d'une première stabilité relative à un état équilibré de l'utilisateur conformément à une longueur de la trajectoire et d'une seconde stabilité relative à un état équilibré de l'utilisateur conformément à une valeur maximale de fluctuation vers la première direction et à une valeur maximale de fluctuation vers la seconde direction.

**5.** Dispositif de détection de fluctuation selon la revendication 1, dans lequel

l'unité de retrait (130) est configurée pour soustraire une valeur prescrite de la première quantité de fluctuation et de la seconde quantité de fluctuation lorsque la respiration de l'utilisateur est détectée pendant la mesure.

**6.** Dispositif de détection de fluctuation selon la revendication 1, dans lequel

l'unité de retrait (130) est configurée pour soustraire une valeur conformément à une valeur d'amplitude de la bande de fréquences particulière à partir de la première quantité de fluctuation et de la seconde quantité de fluctuation lorsque la respiration de l'utilisateur est détectée pendant la mesure.

**7.** Procédé de détection de fluctuation qui détecte la fluctuation d'un corps d'un utilisateur, le procédé de détection de fluctuation comprenant :

la mesure d'une première distance qui est une distance par rapport à un objet immobile existant dans une première direction et d'une seconde distance qui est une distance par rapport à un objet immobile existant dans une seconde direction différente de la première direction (S1205) ;

le calcul d'une première quantité de fluctuation qui est une quantité changeante de la première distance et d'une seconde quantité de fluctuation qui est une quantité changeante de la seconde distance (S1404) ;

la détection d'une respiration de l'utilisateur (S1307) ; et

le retrait d'une quantité changeante de la première distance provoquée par la respiration de l'utilisateur à partir de la première quantité de fluctuation et le retrait d'une quantité changeante de la seconde distance provoquée par la respiration à partir de la seconde quantité de fluctuation, lorsque la respiration de l'utilisateur est détectée pendant la mesure (S1406), lorsqu'une valeur d'amplitude d'une bande de fréquences particulière parmi des bandes de fréquences obtenues en exécutant la transformée de Fourier sur les données sonores de la respiration de l'utilisateur est égale ou supérieure à une valeur définie.

**8.** Programme qui, lorsqu'il est exécuté par un dispositif de détection de fluctuation, conduit le dispositif de détection de fluctuation à mettre en oeuvre le procédé selon la revendication 7.

170

STILL OBJECT

SECOND
DISTANCE

SECOND
DIRECTION | FIRST
DIRECTION

160

FIRST
DISTANCE

STILL
OBJECT

150

USER

100

110 — MEASUREMENT
UNIT

120 — CALCULATION
UNIT

130 — REMOVAL UNIT

F I G. 1

200

502          503

501

501b

501a

z

x

y          250

F I G. 2

FACE

300

200

x

z

350

y

F I G. 3

F I G. 4

F I G. 5

600

| MEASUREMENT NUMBER | ELAPSED TIME | DISTANCE Lx [mm] | DISTANCE Lz [mm] | BREATHING FLAG |
|---|---|---|---|---|
| 0 | 00 HOURS 0 MINUTES 00.000 SECONDS | 50 | 60 | 0 |
| 1 | 00 HOURS 0 MINUTES 00.200 SECONDS | 30 | 20 | 0 |
| ... | ... | ... | ... | ... |
| n-1 | 00 HOURS 01 MINUTES 22.800 SECONDS | 40 | 53 | 1 |
| n | 00 HOURS 01 MINUTES 23.000 SECONDS | 56 | 63 | 1 |
| ... | ... | ... | ... | ... |

F I G. 6

700

| TRAJECTORY CHANGING AMOUNT $\Delta D$ [mm] | MAXIMUM FLUCTUATION CHANGING AMOUNT Xmax [mm] | MINIMUM FLUCTUATION CHANGING AMOUNT Xmin [mm] | MAXIMUM FLUCTUATION CHANGING AMOUNT Zmax [mm] | MINIMUM FLUCTUATION CHANGING AMOUNT Zmin [mm] |
|---|---|---|---|---|
| 45 | 0 | −20 | 0 | −40 |
| ... | ... | ... | ... | ... |
| ... | ... | ... | ... | ... |
| 39 | 26 | −23 | 31 | −40 |
| 19 | 26 | −23 | 31 | −40 |
| ... | ... | ... | ... | ... |
| | | | | |

F I G. 7

800

| FLUCTUATION TRAJECTORY LENGTH D[mm] | BODY BALANCED STATE DETERMINATION |
|---|---|
| EQUAL TO OR GREATER THAN 1000mm | BALANCED STATE OF BODY IS UNSTABLE CONTINUOUSLY (STAGE A) |
| EQUAL TO OR GREATER THAN 500mm AND SMALLER THAN 1000mm | BALANCED STATE OF BODY IS A LITTLE UNSTABLE CONTINUOUSLY (STAGE B) |
| EQUAL TO OR GREATER THAN 200mm AND SMALLER THAN 500mm | BALANCED STATE OF BODY IS A LITTLE STABLE CONTINUOUSLY (STAGE C) |
| SMALLER THAN 200mm | BALANCED STATE OF BODY IS STABLE CONTINUOUSLY (STAGE D) |

F I G. 8

900

| MAXIMUM FLUCTUATION AMOUNT (Z AXIS DIRECTION) [mm] / MAXIMUM FLUCTUATION AMOUNT (X AXIS DIRECTION) [mm] | SMALLER THAN 30mm | EQUAL TO OR GREATER THAN 30mm AND SMALLER THAN 100mm | EQUAL TO OR GREATER THAN 100mm |
|---|---|---|---|
| SMALLER THAN 30mm | BALANCED STATE IS STABLE (STAGE 1) | BALANCED STATE IS A LITTLE UNSTABLE TO FORWARD AND BACKWARD DIRECTIONS (STAGE 2) | BALANCED STATE IS UNSTABLE TO FORWARD AND BACKWARD DIRECTIONS (STAGE 5) |
| EQUAL TO OR GREATER THAN 30mm AND SMALLER THAN 100mm | BALANCED STATE IS A LITTLE UNSTABLE TO RIGHT AND LEFT DIRECTIONS (STAGE 3) | BALANCED STATE IS A LITTLE UNSTABLE TO FORWARD AND BACKWARD DIRECTIONS AND RIGHT AND LEFT DIRECTIONS (STAGE 4) | BALANCED STATE IS UNSTABLE TO FORWARD AND BACKWARD DIRECTIONS AND A LITTLE UNSTABLE TO RIGHT AND LEFT DIRECTIONS (STAGE 7) |
| EQUAL TO OR GREATER THAN 100mm | BALANCED STATE IS UNSTABLE TO RIGHT AND LEFT DIRECTIONS (STAGE 6) | BALANCED STATE IS UNSTABLE TO RIGHT AND LEFT DIRECTIONS AND A LITTLE UNSTABLE TO FORWARD AND BACKWARD DIRECTIONS (STAGE 8) | BALANCED STATE IS UNSTABLE TO RIGHT AND LEFT DIRECTIONS AND FORWARD AND BACKWARD DIRECTIONS (STAGE 9) |

F I G. 9

/1000

| | STAGE A | STAGE B | STAGE C | STAGE D |
|---|---|---|---|---|
| STAGE 1 | ALWAYS FLUCTUATING MINUTELY | INTERMITTENTLY FLUCTUATING MINUTELY | FLUCTUATING MINUTELY IN SHORT TERM | BALANCED STATE IS ALWAYS VERY GOOD |
| STAGE 2 | BALANCED STATE IS ALWAYS UNSTABLE TO FORWARD AND BACKWARD DIRECTIONS | BALANCED STATE IS INTERMITTENTLY UNSTABLE TO FORWARD AND BACKWARD DIRECTIONS | BALANCED STATE IS UNSTABLE TO FORWARD AND BACKWARD DIRECTIONS IN SHORT TERM | BALANCED STATE IS SOMETIMES UNSTABLE TO FORWARD AND BACKWARD DIRECTIONS |
| STAGE 3 | BALANCED STATE IS ALWAYS UNSTABLE TO RIGHT AND LEFT DIRECTIONS | BALANCED STATE IS INTERMITTENTLY UNSTABLE TO RIGHT AND LEFT DIRECTIONS | BALANCED STATE IS UNSTABLE TO RIGHT AND LEFT DIRECTIONS IN SHORT TERM | BALANCED STATE IS SOMETIMES UNSTABLE TO RIGHT AND LEFT DIRECTIONS |
| STAGE 4 | BALANCED STATE IS ALWAYS UNSTABLE TO FORWARD AND BACKWARD DIRECTIONS | BALANCED STATE IS INTERMITTENTLY UNSTABLE TO RIGHT AND LEFT DIRECTIONS | BALANCED STATE IS UNSTABLE TO RIGHT AND LEFT DIRECTIONS IN SHORT TERM | BALANCED STATE IS SOMETIMES UNSTABLE TO RIGHT AND LEFT DIRECTIONS |
| STAGE 5 | BALANCED STATE IS ALWAYS VERY UNSTABLE TO FORWARD AND BACKWARD DIRECTIONS | BALANCED STATE IS INTERMITTENTLY VERY UNSTABLE TO FORWARD AND BACKWARD DIRECTIONS | BALANCED STATE IS VERY UNSTABLE TO FORWARD AND BACKWARD DIRECTIONS IN SHORT TERM | BALANCED STATE IS SOMETIMES VERY UNSTABLE TO FORWARD AND BACKWARD DIRECTIONS |
| STAGE 6 | BALANCED STATE IS ALWAYS VERY UNSTABLE TO RIGHT AND LEFT DIRECTIONS | BALANCED STATE IS INTERMITTENTLY VERY UNSTABLE TO RIGHT AND LEFT DIRECTIONS | BALANCED STATE IS VERY UNSTABLE TO RIGHT AND LEFT DIRECTIONS IN SHORT TERM | BALANCED STATE IS SOMETIMES VERY UNSTABLE TO RIGHT AND LEFT DIRECTIONS |
| STAGE 7 | BALANCED STATE IS ALWAYS A LITTLE UNSTABLE TO RIGHT AND LEFT DIRECTIONS AND VERY UNSTABLE TO FORWARD AND BACKWARD DIRECTIONS | BALANCED STATE IS INTERMITTENTLY A LITTLE UNSTABLE TO RIGHT AND LEFT DIRECTIONS AND VERY UNSTABLE TO FORWARD AND BACKWARD DIRECTIONS | BALANCED STATE IS A LITTLE UNSTABLE TO RIGHT AND LEFT DIRECTIONS IN SHORT TERM AND VERY UNSTABLE TO FORWARD AND BACKWARD DIRECTIONS | BALANCED STATE IS SOMETIMES A LITTLE UNSTABLE TO RIGHT AND LEFT DIRECTIONS AND VERY UNSTABLE TO FORWARD AND BACKWARD DIRECTIONS |
| STAGE 8 | BALANCED STATE IS ALWAYS A LITTLE UNSTABLE TO FORWARD AND BACKWARD DIRECTIONS AND VERY UNSTABLE TO RIGHT AND LEFT DIRECTIONS | BALANCED STATE IS INTERMITTENTLY A LITTLE UNSTABLE TO FORWARD AND BACKWARD DIRECTIONS AND IS VERY UNSTABLE TO RIGHT AND LEFT DIRECTIONS | BALANCED STATE IS A LITTHLE UNSTABLE TO FORWARD AND BACKWARD DIRECTIONS IN SHORT TERM AND VERY UNSTABLE TO RIGHT AND LEFT DIRECTIONS | BALANCED STATE IS SOMETIMES A LITTLE UNSTABLE TO FORWARD AND BACKWARD DIRECTIONS AND VERY UNSTABLE TO RIGHT AND LEFT DIRECTIONS |
| STAGE 9 | BALANCED STATE IS ALWAYS VERY UNSTABLE TO ALL DIRECTIONS | BALANCED STATE IS INTERMITTENTLY VERY UNSTABLE TO ALL DIRECTIONS | BALANCED STATE IS VERY UNSTABLE TO ALL DIRECTIONS IN SHORT TERM | BALANCED STATE IS SOMETIMES VERY UNSTABLE TO ALL DIRECTIONS |

F I G. 1 0

START — S1100

OBTAIN ACCELERATION OF FIRST COORDINATE SYSTEM — S1101

S1102
ARE Y AXIS DIRECTIONS OF FIRST COORDINATE SYSTEM AND GRAVITY ACCELERATION DIRECTIONS IDENTICAL?

NO

YES

START OBTAINMENT OF AUDIO DATA — S1103

S1104
IS THERE TERMINATION REQUEST?

YES

NO — S1105

ACTIVATE THREAD 1 — S1105

ACTIVATE THREAD 2 — S1106

ACTIVATE THREAD 3 — S1107

STOP OBTAINMENT OF AUDIO DATA AND EMISSION FROM OPTICAL SENSOR — S1108

CALCULATE TRAJECTORY LENGTH D OF FLUCTUATION — S1109

CALCULATE MAXIMUM FLUCTUATION AMOUNTS Lxmax AND Lzmax — S1110

DETERMINE BALANCED STATE OF BODY — S1111

REPORT DETERMINATION RESULT TO USER — S1112

END — S1113

F I G. 1 1

START OF THREAD 1 — S1200

IS THERE TERMINATION REQUEST? — S1201

YES → END — S1202

NO

HAS PRESCRIBED PERIOD OF TIME ELAPSED? — S1203

NO

YES

MEASURE REFLECTION TIME OF LIGHT — S1204

CALCULATE DISTANCES Lx AND Lz TO STILL OBJECT — S1205

F I G.  1 2

START OF THREAD 2 — S1300

S1301

IS THERE TERMINATION REQUEST? — YES → S1302 — END

NO

S1303

HAS PRESCRIBED PERIOD OF TIME ELAPSED? — NO

YES

FOURIER TRANSFORM ON AUDIO DATA — S1304

OBTAIN DATA BETWEEN 0.25Hz AND 0.33Hz — S1305

INVERSE FOURIER TRANSFORM — S1306

S1307

IS AMPLITUDE EQUAL TO OR GREATER THAN FIXED VALUE? — NO

YES — S1308

SET BREATHING FLAG TO 1

S1309

SET BREATHING FLAG TO 0

F I G. 1 3

F I G. 1 4

FACE

WALL

1501

200

WALL

1502

300

x

350

z

y

F I G. 1 5

F I G. 1 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004344468 A
- JP 2008073267 A
- GB 2438167 A
- US 20060251334 A